(19) Europäisches Patentamt
European Patent Office
Office européen des brevets

(11) Veröffentlichungsnummer: **0 195 744**
**B1**

(12) **EUROPÄISCHE PATENTSCHRIFT**

(45) Veröffentlichungstag der Patentschrift: **16.05.90**

(21) Anmeldenummer: **86810115.5**

(22) Anmeldetag: **05.03.86**

(51) Int. Cl.[5]: **C 07 C 53/21**, C 07 C 51/15, C 07 C 327/06, C 07 C 313/02, C 07 F 3/06

(54) **Verfahren zur Herstellung von Trifluordichlorethyl substituierten Säuren und Zinkverbindungen.**

(30) Priorität: **11.03.85 CH 1090/85**

(43) Veröffentlichungstag der Anmeldung:
**24.09.86 Patentblatt 86/39**

(45) Bekanntmachung des Hinweises auf die Patenterteilung:
**16.05.90 Patentblatt 90/20**

(84) Benannte Vertragsstaaten:
**CH DE FR GB IT LI NL**

(56) Entgegenhaltungen:
**EP-A-0 165 135**
**DE-A-2 848 197**
**FR-A-2 342 950**
**FR-A-2 374 287**

(73) Patentinhaber: **CIBA-GEIGY AG**
**Klybeckstrasse 141**
**CH-4002 Basel (CH)**

(72) Erfinder: **Lang, Robert Werner, Dr.**
**Hagenbachweg 10**
**CH-4133 Pratteln (CH)**
Erfinder: **Klingert, Bernd, Dr.**
**Möndenweg 87**
**D-7851 Inzlingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 195 744 B1

Courier Press, Leamington Spa, England.

**Beschreibung**

Die vorliegende Erfindung betrifft ein Verfahren zur Herstellung von 2,2,2-Trifluor-1,1-dichlorethylcarbonsäure, -thiocarbonsäure und -sulfinsäure unter Verwendung von 2,2,2-Trifluor-1,1-dichlorzinkchlorid-Lösungsmitteladdukten, und diese Zinkchlorid-Lösungsmitteladdukte.

Aus der US—PS 3,290,333 sind 2,2,2-Trifluor-1,1-dichlorethylzinkchlorid-Lösungsmitteladdukte mit Aethern, z.B. Dioxan und Tetrahydrofuran, bekannt. A. Posta und O. Paleta beschreiben in Collection Czechoslov. Chem. Commun., Vol. 37, S. 3946—3949 (1972), dass das 2,2,2-Trifluor-1,1-dichlorzinkchlorid-Addukt mit Dioxan bei der Umsetzung mit einem Acetylchlorid im Gegensatz zur gleichen Reaktion mit einem Perfluoralkylzinkjodid nicht das erwartete Keton liefert. Eigene Untersuchungen ergaben, dass die beschriebenen Zinkchlorid-Addukte auch nicht mit Kohlendioxid zu reagieren vermögen. Dieses Ergebnis wird in J.C.S. Chem. Comm., S. 885—886 (1976) bestätigt, wo beschrieben ist, dass selbst Perfluoralkylzinkjodide nicht mit Kohlendioxid reagieren. Im J. Fluorine Chemistry 22, S. 585 (1983) wird beschrieben, dass Perfluoralkyljodide in Gegenwart von Zink und Dimethylformamid als Lösungsmittel mit $CO_2$ dann zu Perfluoralkylcarbonsäuren umgesetzt werden, wenn die Reaktion unter Einwirkung von Ultraschall durchgeführt wird.

Ein Gegenstand vorliegender Erfindung ist ein Verfahren zur Herstellung von Trifluordichlorethyl substituierten Säuren der Formel I

$$F_3C—CCl_2—X \qquad (I),$$

worin X für —COOH, —C(S)OH oder —$SO_2$H steht, durch Umsetzung einer metallorganischen Verbindung mit $CO_2$, COS oder $SO_2$ in Gegenwart eines inerten Lösungsmittels und anschliessender Hydrolyse, das dadurch gekennzeichnet ist, dass man als metallorganische Verbindung eine Zinkverbindung der Formel II

$$CF_3CCl_2ZnCl \cdot yL \qquad (II)$$

verwendet, worin y die Zahl 1 oder 2 ist und L ein Lösungsmittelligand aus der Gruppe der N-disubstituierten Säureamide, der N-substituierten Lactame und der organischen Sulfoxide bedeutet.

Beim erfindungsgemässen Verfahren wird vorzugsweise $SO_2$ und besonders $CO_2$ zur Herstellung von 2,2,2-Trifluor-1,1-dichlorsulfinsäure bzw. 2,2,2-Trifluor-1,1-dichlorpropionsäure eingesetzt.

Die Zinkverbindungen der Formel II sind neu und ebenfalls ein Gegenstand vorliegender Erfindung.

In Formel II stellt L in seiner Bedeutung als N-disubstituiertes Säureamid bevorzugt ein Carbonsäureamid dar, das insbesondere der Formel

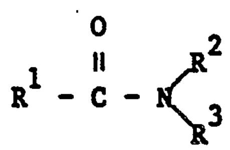

entspricht, worin $R^1$ für ein Wasserstoffatom, unsubstituiertes oder mit Halogen, besonders Fluor oder Chlor, substituiertes Alkyl mit 1 bis 12, besonders 1 bis 4 C-Atomen, unsubstituiertes oder mit $C_1$—$C_4$-Alkyl oder Halogen substituiertes Cycloalkyl mit 4 bis 7 Ringkohlenstoffatomen, Alkenyl mit 2 bis 12, besonders 2 bis 4 C-Atomen, Phenyl, Benzyl oder —$NR^2R^3$ steht, und $R^2$ und $R^3$ unabhängig voneinander $C_1$—$C_{12}$-Alkyl, Cycloalkyl mit 5 oder 6 Ringkohlenstoffatomen, oder $R^2$ und $R^3$ zusammen gegebenenfalls durch —O—, —S— oder —$NR^4$— ($R^4$ gleich $C_1$—$C_4$-Alkyl) unterbrochenes Tetra- oder Pentamethylen bedeuten.

$R^1$ steht besonders bevorzugt für ein Wasserstoffatom oder Methyl.

$R^2$ und $R^3$ bedeuten besonders Methyl oder Ethyl.

Beispiele für Säureamide sind Dimethylformamide, Diethylformamid, Dimethylacetamid, Tetramethylharnstoff und N-Formylpyrrolidin. Besonders bevorzugt ist Dimethylformamid.

L in seiner Bedeutung als N-substituiertes Lactam entspricht bevorzugt der Formel

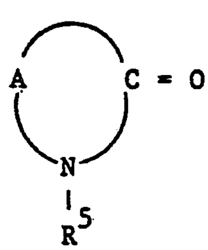

worin A für Di-, Tri-, Tetra- oder Pentamethylen steht, und $R^5$ $C_1$—$C_{12}$, besonders $C_1$—$C_4$-Alkyl, Cyclohexyl oder Cyclopentyl bedeutet. $R^5$ ist besonders Methyl oder Ethyl, Beispiele für solche Lactame sind N-Methylpropiolactam, N-Methylpyrrolidon, N-Ethylpyrrolidon, N-Methylpiperidinon, N-Methyl-ε-

caprolactam. Bevorzugt ist N-Methylpyrrolidon.

L in seiner Bedeutung als organisches Sulfoxid entspricht bevorzugt der Formel

$$R^6-\overset{\overset{\displaystyle O}{\|}}{S}-R^7,$$

worin $R^6$ und $R^7$ unabhängig voneinander $C_1-C_{12}$-, besonders $C_1-C_4$-Alkyl oder $R^6$ und $R^7$ zusammen Tetra- oder Pentamethylen bedeuten. Beispiele sind Dimethyl-, Methylethyl-, Diethylsulfoxid, Tetramethylensulfoxid und Pentamethylensulfoxid.

Von den Zinkverbindungen der Formel II sind jene bevorzugt, in denen L ein N-disubstituiertes Carbonsäureamid ist. In Formel II ist y bevorzugt 2. Eine besonders bevorzugte Zinkverbindung ist $CF_3CCl_2ZnCl \cdot 2$ Dimethylformamid.

Die Herstellung der Zinkverbindungen der Formel II erfolgt in an sich bekannter Weise durch die direkte Umsetzung von Zink, das zweckmässig als Zinkstaub vorliegt, mit 1,1,1-Trifluor-2,2,2-trichlorethan unter Luft- und Feuchtigkeitsausschluss und in einem inerten Lösungsmittel. Vorteilhaft wird die Reaktionsmischung gekühlt. Die erfindungsgemässen Zinkverbindungen können dann in üblicher Weise durch Entfernen des Lösungsmittel oder Kristallisation isoliert werden.

In einem anderen Herstellverfahren kann man so vorgehen, dass man die bekannten Etheraddukte von $CF_3CCl_2ZnCl$ (vgl. US—PS 3 290 333) in einem Lösungsmittel L löst und gegebenenfalls erwärmt bis etwa 100°C, wobei sich durch Ligandenaustausch die erfindungsgemässen Addukte bilden, die dann in bekannter Weise isoliert werden können.

Bei den erfindungsgemässen Zinkverbindungen handelt es sich um kristalline Verbindungen die unter Luft- und Feuchtigkeitsausschluss stabil sind. Die erfindungsgemässen Zinkverbindungen haben sich überraschend gegenüber $CO_2$, COS und $SO_2$ als reaktiv erwiesen, wobei ein Arbeiten mit Ultraschall möglich, aber nicht notwendig ist. Bei der Reaktion werden die gewünschten Säuren in guten Ausbeuten und hoher Reinheit gebildet. Das Verfahren ist durch die Verwendung billiger Ausgangsprodukte besonders wirtschaftlich.

Das erfindungsgemässe Verfahren wird vorzugsweise in einem inerten polaren, aprotischen Lösungsmittel durchgeführt. Solche Lösungsmittel sind z.B. besonders Ether, tertiäre Amine, Sulfone und die dem Liganden L entsprechenden Lösungsmittel. Beispiele sind Dimethyl-, Diethyl-, Dibutylether, Dioxan, Tetrahydrofuran, Ethylenglykoldimethyl-oder-diethyläther, Diethylenglykoldimethylether, Triethylenglykoldimethylether, Dimethylsulfon, Tetra- oder Pentamethylensulfon, Trimethyl-, Triethyl-, Methyldiethyl-, Tripropyl-, Tributylamin, N-Methylmorpholin, N-Methylpiperidin, N-Methylpyrrolidin, Pyridin, N-Methylpyrrol.

Bevorzugt handelt es sich bei dem inerten Lösungsmittel um ein N-disubstituiertes Säureamid, ein N-substituiertes Lactam oder ein organisches Sulfoxid und insbesondere sind der Ligand L und das Lösungsmittel identisch. Besonders bevorzugt ist das Lösungsmittel Dimethylformamid. In einer anderen Ausführungsform werden Zinkverbindungen der Formel II eingesetzt, worin y für 2 steht und L für Dimethylformamid steht.

In einer vorteilhaften Ausführungsform wird ein dem Liganden L entsprechendes Lösungsmittel verwendet und die Zinkverbindung II in situ durch die Umsetzung von $CF_3CCl_3$ mit Zink hergestellt und darauf die Reaktion mit $CO_2$, COS oder $SO_2$ durchgeführt.

Das erfindungsgemässe Verfahren wird zweckmässig bei einer Temperatur von 0 bis 50°C durchgeführt, vorzugsweise bei Raumtemperatur. Das verfahren kann bei Normaldruck oder Ueberdruck durchgeführt werden, z.B. in Autoklaven, wobei der Druck bis etwa 100, bevorzugt bis zu 50 bar betragen kann.

Das Reaktionsgemisch wird zur Isolierung der Säuren hydrolytisch aufgearbeitet. Die Hydrolyse wird vorteilhaft mit verdünnten Mineralsäuren vorgenommen, z.B. Salzsäure oder Schwefelsäure, die zweckgemäss zur internen Kühlung mit Eis vermischt werden. Nach der Hydrolyse können die gebildeten Säuren mit einem geeigneten Lösungsmittel extrahiert werden, z.B. Diethylether. Nach Entfernen des Lösungsmittels durch Destillation erhält man die gewünschten Säuren, die durch Destillation noch gereinigt werden können.

Es hat sich als zweckmässig erwiesen, dass man vor der Hydrolyse des Reaktionsgemisches die gebildeten Zinksalze isoliert, die als kristalline Feststoffe anfallen und durch Kristallisation leicht erhältlich sind. Hierzu wird das verwendete Lösungsmittel entfernt und der Rückstand aus einem geeigneten Lösungsmittel umkristallisiert. Die Zinksalze können den Formeln

$$(CF_3CCl_2Y)_2Zn \cdot yL \text{ oder } (CF_3CCl_2Y)ZnCl \cdot yL$$

entsprechen. Y steht für —C(O)O—, —C(S)O— oder —S(O)O—. Durch Hydrolyse können aus den Zinksalzen die Säuren erhalten werden.

Die erfindungsgemäss hergestellten Säuren sich z.B. als Veresterungskatalysatoren oder zur Herstellung von oberflächenaktiven Substanzen, von Oel und Wasser abweisenden Imprägniermitteln und

von Pflanzenschutzmitteln (vgl. DE—OS 1 900 758).

Die nachfolgenden Beispiele erläuteren die Erfindung näher. Die Reaktionen werden unter Feuchtigkeitsausschluss und Schutzgasatmosphäre (Stickstoff oder Argon) durchgeführt.

Beispiel 1

Herstellung von $CF_3CCl_2ZnCl\ (LM)_n$-Komplexen

In einem 1 l Dreihalsrundkolben werden 65,4 g (1 Mol) Zinkstaub (aktiviert nach Fieser & Fieser) im Lösungsmittel (LM) suspendiert und langsam mit 188 g (1 Mol) $CF_3CCl_3$ versetzt (LM-Menge; Reaktionszeit und Reaktionstemperatur siehe Tabelle 1). Anschliessend wird die Reaktionsmischung bei der Reaktionstemperatur über Filterflockenmasse "Selecta" filtriert, wobei nach Erkalten oder Kühlen des Filtrats die Komplexe in kristalliner Form ausfallen. Im Falle von Dimethylformamid (DMF) als LM wird durch Zusatz von Diethyläther ($Et_2O$) ausgefällt. Die Komplexe werden zur weiteren Reinigung aus dem jeweiligen LM umkristallisiert bzw. im Falle von LM = DMF mit $Et_2O$ umgefällt. Das LM wird abdekantiert und der farblos kristalline Rückstand im Vakuum getrocknet. Die Ausbeuten betragen 70—80%. Die Ergebnisse sind in Tabelle 1 zusammengefasst.

Tabelle 1

| Komplex | LM-Menge | Reaktionszeit (Stunden) | Reaktionstemperatur | Schmelzpunkt (°C) |
|---|---|---|---|---|
| $CF_3CCl_2ZnCl$ (Dioxan) | 500 ml | 3 | 101°C | 173 |
| $CF_3CCl_2ZnCl$ ($Et_2O$) | 800 ml | 20 | Raumtemperatur | 105 |
| $CF_3CCl_2ZnCl$ $(THF)_2$* | 800 ml | 3 | " | 135 |
| $CF_3CCl_2ZnCl$ $(DME)_1$** | 800 ml | 3 | " | 68 |
| $CF_3CCl_2ZnCl$ $(DMF)_2$ | 500 ml | 2 | " | 67 |

*) Tetrahydrofuran, **) Dimethoxyethan

Beispiel 2

Herstellung von $CF_3CCl_2ZnCl\ (DMF)_2$ durch Ligand-Austauschreaktion

In einen 100 ml dreihalsrundkolben werden 4,0 g (12 mmol) $CF_3CCl_2ZnCl\ (Et_2O)$ bei Raumtemperatur in 50 ml $Et_2O$ gelöst. Es werden langsam 2 ml (26 mmol) DMF unter starkem Rühren zugegeben. Nach kurzer Zeit bildet sich ein Zweiphasensystem, wobei sich der DMF-Komplex als untere Phase in öliger Form absetzt. Nach wenigen Minuten beginnt dieser zu kristallisieren. Es wird abdekantiert, mit $Et_2O$ gewaschen und anschliessend im Vakuum getrocknet (Ausbeute > 95%).

Beispiel 3

Herstellung von 1,1,1-Trifluor-2,2-dichlorpropionsäure im Eintopfverfahren

In einem 1 l Dreihalsrundkolben werden 65,4 g (1 Mol) Zinkstaub (aktiviert nach Fieser & Fieser) in 500 ml DMF suspendiert und bei Raumtemperatur langsam mit 188 g (≙ 120 ml: 1 mol) frisch über einem Molekularsieb getrocknetem und anschliessend destilliertem $CF_3CCl_3$ versetzt. Nach wenigen Minuten beginnt das Zink in Lösung zu gehen, wobei sich die Reaktionsmischung erwärmt. Die Reaktionstemperatur wird mit externer Kühlung unter 30° gehalten. Anschliessend wird 1 Stunde nachgerührt und unter Schutzgas über Filterflockenmasse "Selecta" (Fa. Schleicher & Schüll) filtriert. In das rotbraune, klare Filtrat wird unter kräftigem Rühren über eine Fritte während 6 Stunden $CO_2$-Gas eingeleitet, wobei anfänglich eine starke Absorption auftritt Anschliessend wird die Lösung auf eine Mischung aus 500 ml 10%-ige wässrige HCl/300 g Eis gegossen und 5x mit je 300 ml $Et_2O$ extrahiert. Es wird mit 200 ml 2%-iger wässriger HCl nachgewaschen, über $MgSO_4$ getrocknet und am Rotationsverdampfer bei Raumtemperatur eingeengt. Die verbleibende braune Flüssigkeit wird bei 26 mbar destilliert, wobei die Säure bei 62—64°C als farblose Flüssigkeit übergeht (Ausbeute 50%). Die reine Säure erstarrt bei Raumtemperatur und hat einen Schmelzpunkt von 25—30°C.

Beispiel 4

Herstellung von 1,1,1-Trifluor-2,2-dichloropropionsäure ausgehend von isolierten $CF_3CCl_2ZnCl(LM)_n$-Komplexen

In einem 1 l Dreihalsrundkolben werden 1 Mol der Komplexe (siehe Tabelle 1) bei Raumtemperatur in 500 ml DMF gelöst und gemäss Beispiel 3 mit $CO_2$-Gas versetzt und aufgearbeitet. Die Ausbeuten an isolierter Säure betragen 35—50%.

Beispiel 5

Herstellung von $(CF_3CCl_2COO)_2Zn\ (DMF)$

In einem 90 ml Glasautoklav werden 16,4 g (50 mmol) $CF_3CCl_2ZnCl\ (Et_2O)$ bei Raumtemperatur (RT) und unter kräftigem Rühren mit 35 ml DMF versetzt. Anschliessend wird $CO_2$-Gas aufgepresst und während 24 Stunden bei 5,0 bar und 40°C konstant gehalten. Zum Schluss wird im Hockvakuum bei Raumtemperatur das Lösungsmittel abgezogen und der gelbe, harzige Rückstand mit Aether digeriert. Der verbleibende Feststoff wird aus Aceton umkristallisiert. Man erhält 8,2 g (62%) farblose Nadeln vom Smp. 188°C. Die Hydrolyse gemäss Beispiel 3 ergibt die freie Säure.

Beispiel 6

Herstellung von $CF_3CCl_2SO_2ZnCl\ (DMF)_2$

In einem analogen Ansatz wie in Beispiel 5, wird anstelle von $CO_2$-Gas mit $SO_2$ während 24 Stunden bei 40°C ein Druck von 2,2 bar eingestellt. Nach Abziehen des Lösungsmittels unter vermindertem Druck wird der verbleibende Feststoff mit Aether digeriert. Man erhält 12,2 g (53%) farblose Kristalle vom Smp. 100—110°C. Die Hydrolyse gemäss Beispiel 3 ergibt die freie Sulfinsäure.

**Patentansprüche**

1. Verfahren zur Herstellung von Trifluordichlorethyl substituierten Säuren der Formel

$$F_3C—CCl_2—X \qquad (I)$$

worin X für —COOH, —C(S)OH oder —$SO_2$H steht, durch Umsetzung einer metallorganischen Verbindung mit $CO_2$, COS oder $SO_2$ in Gegenwart eines inerten Lösungsmittels und anschliessender Hydrolyse, dadurch gekennzeichnet, dass man als metallorganische Verbindung eine Zinkverbindung der Formel II

$$CF_3CCl_2ZnCl \cdot yL \qquad (II)$$

verwendet, worin y die Zahl 1 oder 2 ist und L ein Lösungsmittelligand aus der Gruppe der N-disubstituierten Säureamide, der N-substituierten Lactame und der organischen Sulfoxide bedeutet.

2. Verfahren gemäss Anspruch 1, dadurch gekennzeichnet, dass es sich bei dem inerten Lösungsmittel um ein polares aprotisches Lösungsmittel handelt.

3. Verfahren gemäss Anspruch 2, dadurch gekennzeichnet, dass das Lösungsmittel ein N-disubstituiertes Säureamid, ein N-substituiertes Lactam oder ein organisches Sulfoxid ist.

4. Verfahren gemäss Anspruch 1, dadurch gekennzeichnet, dass der Lösungsmittelligand L und das inerte Lösungsmittel identisch sind.

5. Verfahren gemäss Anspruch 1, dadurch gekennzeichnet, dass y für 2 steht und L Dimethylformamid bedeutet.

6. Verfahren gemäss Anspruch 1, dadurch gekennzeichnet, dass die Umsetzung in einem dem Lösungsmittelliganden L entsprechenden Lösungsmittel durchgeführt wird und die Zinkverbindung II in situ durch die Umsetzung von $CF_3$—$CCl_3$ mit Zink hergestellt wird.

7. Verfahren gemäss Anspruch 1, dadurch gekennzeichnet, dass es bei einer Temperatur von 0 bis 50°C durchgeführt wird.

8. Verfahren gemäss Anspruch 1, dadurch gekennzeichnet, dass die Hydrolyse des Reaktionsgemisches mit einer verdünnten Mineralsäure durchgeführt wird.

9. Verfahren gemäss Anspruch 1, dadurch gekennzeichnet, dass es bei erhöhtem Druck durchgeführt wird.

10. Verfahren gemäss Anspruch 1, dadurch gekennzeichnet, dass man vor der Hydrolyse die gebildeten Zinksalze isoliert

11. Zinkverbindung der Formel II

$$CF_3CCl_2ZnCl\cdot yL \qquad (II),$$

worin y die Zahl 1 oder 2 ist und L ein Lösungsmittelligand aus der Gruppe der N-disubstituierten Säureamide, der N-substituierten Lactame und der organischen Sulfoxide bedeutet.

12. Zinkverbindung gemäss Anspruch 11, dadurch gekennzeichnet, dass L ein N-disubstituiertes Carbonsäureamid ist.

13. Zinkverbindung gemäss Anspruch 11, dadurch gekennzeichnet, dass y für 2 steht.

14. Zinkverbindung gemäss Anspruch 11 der Formel $CF_3CCl_2ZnCl\cdot 2$ Dimethylformamid.

**Revendications**

1. Procédé pour préparer des acides à radical trifluoro-dichloréthyle répondant à la formule I:

$$F_3C—CCl_2—X \qquad (I)$$

dans laquelle X représente un radical —COOH, —C(S)OH ou —$SO_2$H, par réaction d'un composé organométallique avec $CO_2$, COS ou $SO_2$ en présence d'un solvant inerte, puis hydrolyse, procédé caractérisé en ce qu'on utilise, comme composé organométallique, un composé du zinc répondant à la formule II:

$$CF_3CCl_2ZnCl\cdot yL \qquad (II)$$

dans laquelle y représente le nombre 1 ou le nombre 2 et L représente un solvant pris dans l'ensemble constitué par les amides disubstitués à l'azote, les lactames substitués à l'azote et les sulfoxydes organiques.

2. Procédé selon la revendication 1 caractérisé en ce que le solvant inerte est un solvant aprotique polaire.

3. Procédé selon la revendication 2 caractérisé en ce que le solvant est un amide disubstitué à l'azote, un lactame substitué à l'azote ou un sulfoxyde organique.

4. Procédé selon la revendication 1 caractérisé en ce que le coordinat solvant L et le solvant inerte sont identiques.

5. Procédé selon la revendication 1 caractérisé en ce que y est égal à 2 et L représente le diméthylformamide.

6. Procédé selon la revendication 1 caractérisé en ce que la réaction est effectuée dans un solvant correspondant au coordinat solvant L, et le composé du zinc (II) est préparé insitu par réaction de $CF_3CCl_3$ avec du zinc.

7. Procédé selon la revendication 1 caractérisé en ce qu'il est exécuté à une température de 0 à 50°C.

8. Procédé selon la revendication 1 caractérisé en ce que l'hydrolyse du mélange réactionnel est effectuée avec un acide minérale diluée.

9. Procédé selon la revendication 1 caractérisé en ce qu'il est exécuté sous pression élevée.

10. Procédé selon la revendication 1 caractérisé en ce qu'avant d'effectuer l'hydrolyse on isole les sels de zinc formés.

11. Composé du zinc répondant à la formule II:

$$CF_3CCl_2ZnCl\cdot yL \qquad (II)$$

dans laquelle y représente le nombre 1 ou le nombre 2 et L représente un coordinat solvant pris dans l'ensemble constitué par les amides disubstitués à l'azote, les lactames substitués à l'azote et les

sulfoxydes organiques.

12. Composé du zinc selon la revendication 11 caractérisé en ce que L représente un carboxamide disubstitué à l'azote.

13. Composé du zinc selon la revendication 11 caractérisé en ce que y est égal à 2.

14. Composé du zinc selon la revendication 11 qui répond à la formule:

$$CF_3CCl_2ZnCl \cdot 2 \text{ diméthylformamide}.$$

**Claims**

1. A process for the preparation of a trifluorodichloroethyl-substituted acid of the formula

$$F_3C—CCl_2—X \quad (I)$$

in which X is —COOH, —C(S)OH or —$SO_2H$, by reacting an organometal compound with $CO_2$, COS or $SO_2$, in the presence of an inert solvent, and subsequent hydrolysis, which process comprises using a zinc compound of the formula II

$$CF_3CCl_2ZnCl \cdot yL \quad (II)$$

as organometal compound, in which y is 1 or 2 and L is a solvent ligand from the group of the N-disubstituted acid amides, N-substituted lactams and organic sulfoxides.

2. A process according to claim 1, wherein the inert solvent is a polar aprotic solvent.

3. A process according to claim 2, wherein the solvent is an N-disubstituted acid amide, an N-substituted lactam or an organic sulfoxide.

4. A process according to claim 1, wherein the solvent ligand L and the inert solvent are identical.

5. A process according to claim 1, wherein y is 2 and L is dimethylformamide.

6. A process according to claim 1, wherein the reaction is carried out in a solvent corresponding to the solvent ligand L and the zinc compound II is prepared in situ by reacting $CF_3—CCl_3$ with zinc.

7. A process according to claim 1, wherein it is carried out at a temperature from 0 to 50°C.

8. A process according to claim 1, wherein the hydrolysis of the reaction mixture is carried out with a dilute mineral acid.

9. A process according to claim 1, wherein it is carried out under elevated pressure.

10. A process according to claim 1, wherein the zinc salts formed are isolated before the hydrolysis.

11. A zinc compound of the formula II

$$CF_3CCl_2ZnCl \cdot yL \quad (II)$$

in which y is the number 1 or 2 and L is a solvent ligand from the group of the N-disubstituted acid amides, N-substituted lactams and the organic sulfoxides.

12. A zinc compound according to claim 11, wherein L is an N-disubstituted carboxamide.

13. A zinc compound according to claim 11, wherein y is 2.

14. A zinc compound according to claim 11, of the formula $CF_3CCl_2ZnCl \cdot 2$ dimethylformamide.